# EUROPEAN PATENT APPLICATION

(11) **EP 3 656 397 A2**
(43) Date of publication of application: **27.05.2020**
(21) Application number: 18834547.4
(22) Date of filing: 12.09.2018
(51) Int. Cl.: A61K 39/395, A61K 38/17, A61K 38/10, C07K 16/30, C07K 7/08, C07K 14/00, A61P 35/00

(54) **NEW TARGET FOR TREATING CANCER**

(30) Priority: 17.07.2017 CN 201710581764; 17.11.2017 CN 201711146247
(71) Applicant: Tongji University Suzhou Institute Biomedical Research Center, Suzhou, Jiangsu 215000 (CN); Shanghai East Hospital, Shanghai 200120 (CN); Tongji University, Shanghai 200092 (CN)
(72) Inventor: FANG, Jianmin, Suzhou Jiangsu 215000 (CN); GAO, Hua, Shanghai 200120 (CN); CHEN, Guang, Shanghai 200120 (CN); YIN, Yanxin, Suzhou Jiangsu 215000 (CN); GUO, Jia, Suzhou Jiangsu 215000 (CN)
(74) Representative: Wilson, Justin Scott
(86) International application number: PCT/CN2018/105226
(87) International publication number: WO 2019/015696

(57) **Abstract**

The present disclosure relates to a target for treating cancer. Specifically, the present disclosure relates to the use of the extracellular loop 1 (ECL 1) of TM4SF1 as a target for cancer treatment. More specifically, the present disclosure relates to specificity binding in respect of antibodies of the ECL1 of TM4SF1 and peptides of said ECL1.

SEQUENCE LISTING

<110>  TONGJI UNIVERSITY SUZHOU INSTITUTE BIOMEDICAL RESEARCH CENTER, et al

<120>  NEW TARGET FOR TREATING CANCER

<130>  OP1918-EP-0740

<150>  CN 201710581764.8
<151>  2017-07-17

<150>  CN 201711146247.4
<151>  2017-11-17

<160>  18

<170>  PatentIn version 3.2

<210>  1
<211>  609
<212>  DNA
<213>  Homo sapiens

<220>
<221>  gene
<222>  (1)..(609)

<400>  1
atgtgctatg ggaagtgtgc acgatgcatc ggacattctc tggtggggct cgccctcctg      60

tgcatcgcgg ctaatatttt gctttacttt cccaatgggg aaacaaagta tgcctccgaa     120

aaccacctca gccgcttcgt gtggttcttt tctggcatcg taggaggtgg cctgctgatg     180

ctcctgccag catttgtctt cattgggctg gaacaggatg actgctgtgg ctgctgtggc     240

catgaaaact gtggcaaacg atgtgcgatg ctttcttctg tattggctgc tctcattgga     300

attgcaggat ctggctactg tgtcattgtg gcagcccttg gcttagcaga aggaccacta     360

tgtcttgatt ccctcggcca gtggaactac acctttgcca gcactgaggg ccagtacctt     420

ctggatacct ccacatggtc cgagtgcact gaacccaagc acattgtgga atggaatgta     480

tctctgtttt ctatcctctt ggctcttggt ggaattgaat tcatcttgtg tcttattcaa     540

gtaataaatg gagtgcttgg aggcatatgt ggcttttgct gctctcacca acagcaatat     600

gactgctaa                                                             609

<210>  2
<211>  202
<212>  PRT
<213>  Homo sapiens

<220>
<221>  PEPTIDE
<222>  (1)..(202)

<400>  2

Met Cys Tyr Gly Lys Cys Ala Arg Cys Ile Gly His Ser Leu Val Gly
1               5                   10                  15

Leu Ala Leu Leu Cys Ile Ala Ala Asn Ile Leu Leu Tyr Phe Pro Asn
            20                  25                  30

Gly Glu Thr Lys Tyr Ala Ser Glu Asn His Leu Ser Arg Phe Val Trp
        35                  40                  45

Phe Phe Ser Gly Ile Val Gly Gly Gly Leu Leu Met Leu Leu Pro Ala
    50                  55                  60

Phe Val Phe Ile Gly Leu Glu Gln Asp Asp Cys Cys Gly Cys Cys Gly
65                  70                  75                  80

His Glu Asn Cys Gly Lys Arg Cys Ala Met Leu Ser Ser Val Leu Ala
                85                  90                  95

Ala Leu Ile Gly Ile Ala Gly Ser Gly Tyr Cys Val Ile Val Ala Ala
            100                 105                 110

Leu Gly Leu Ala Glu Gly Pro Leu Cys Leu Asp Ser Leu Gly Gln Trp
        115                 120                 125

Asn Tyr Thr Phe Ala Ser Thr Glu Gly Gln Tyr Leu Leu Asp Thr Ser
    130                 135                 140

Thr Trp Ser Glu Cys Thr Glu Pro Lys His Ile Val Glu Trp Asn Val
145                 150                 155                 160

Ser Leu Phe Ser Ile Leu Leu Ala Leu Gly Gly Ile Glu Phe Ile Leu
                165                 170                 175

Cys Leu Ile Gln Val Ile Asn Gly Val Leu Gly Gly Ile Cys Gly Phe
            180                 185                 190

Cys Cys Ser His Gln Gln Gln Tyr Asp Cys
        195                 200

<210>  3
<211>  57
<212>  DNA
<213>  Homo sapiens

<220>
<221>  gene
<222>  (1)..(57)

<400>  3
cccaatgggg aaacaaagta tgcctccgaa aaccacctca gccgcttcgt gtggttc          57

<210>  4
<211>  19
<212>  PRT
<213>  Homo sapiens

<220>
<221>  PEPTIDE
<222>  (1)..(19)

<400>  4

Pro Asn Gly Glu Thr Lys Tyr Ala Ser Glu Asn His Leu Ser Arg Phe
1               5                   10                  15

Val Trp Phe

<210>  5
<211>  75
<212>  DNA
<213>  Homo sapiens

<220>
<221>  gene
<222>  (1)..(75)

<400>  5
ctttactttc ccaatgggga aacaaagtat gcctccgaaa accacctcag ccgcttcgtg      60

tggttctttt ctggc                                                        75

<210>  6
<211>  25
<212>  PRT
<213>  Homo sapiens

<220>
<221>  PEPTIDE
<222>  (1)..(25)

<400>  6

Leu Tyr Phe Pro Asn Gly Glu Thr Lys Tyr Ala Ser Glu Asn His Leu
1               5                   10                  15

Ser Arg Phe Val Trp Phe Phe Ser Gly
            20                  25

<210>  7
<211>  9
<212>  PRT

<213>  Mus musculus

<220>
<221>  PEPTIDE
<222>  (1)..(9)

<400>  7

Gly Tyr Ser Ile Thr Ser Gly Tyr Ser
1               5

<210>  8
<211>  7
<212>  PRT
<213>  Mus musculus

<220>
<221>  PEPTIDE
<222>  (1)..(7)

<400>  8

Ile Arg Ser Ser Gly Thr Thr
1               5

<210>  9
<211>  6
<212>  PRT
<213>  Mus musculus

<220>
<221>  PEPTIDE
<222>  (1)..(6)

<400>  9

Ala Ser Gln Gly Pro Tyr
1               5

<210>  10
<211>  6
<212>  PRT
<213>  Mus musculus

<220>
<221>  PEPTIDE
<222>  (1)..(6)

<400>  10

Gln Asp Ile Ser Asn Tyr
1               5

<210>  11
<211>  3

<212>  PRT
<213>  Mus musculus


<220>
<221>  PEPTIDE
<222>  (1)..(3)

<400>  11

Tyr Thr Ser
1


<210>  12
<211>  9
<212>  PRT
<213>  Mus musculus


<220>
<221>  PEPTIDE
<222>  (1)..(9)

<400>  12

Gln Gln Gly Asn Thr Leu Pro Trp Thr
1               5


<210>  13
<211>  27
<212>  DNA
<213>  Mus musculus


<220>
<221>  gene
<222>  (1)..(27)

<400>  13
ggctactcca tcaccagtgg ttatagc                                          27


<210>  14
<211>  21
<212>  DNA
<213>  Mus musculus


<220>
<221>  gene
<222>  (1)..(21)

<400>  14
atacgctcca gtggtaccac t                                                21


<210>  15
<211>  18
<212>  DNA
<213>  Mus musculus

<220>
<221>  gene
<222>  (1)..(18)

<400>  15
gcaagccaag gaccttac                                                    18


<210>  16
<211>  18
<212>  DNA
<213>  Mus musculus


<220>
<221>  gene
<222>  (1)..(18)

<400>  16
caggacatta gcaattat                                                    18


<210>  17
<211>  9
<212>  DNA
<213>  Mus musculus


<220>
<221>  gene
<222>  (1)..(9)

<400>  17
tacacatca                                                               9


<210>  18
<211>  27
<212>  DNA
<213>  Mus musculus


<220>
<221>  gene
<222>  (1)..(27)

<400>  18
caacagggta atacgcttcc gtggacg                                          27

## Description

### CROSS-REFERENCE TO RELATED APPLICATIONS

The present application claims the priority of Chinese patent application NO. 201710581764.8, entitled "NEW TARGET FOR TREATING CANCER", filed on July 17, 2017, and Chinese patent application NO. 201711146247.4, entitled "NEW TARGET FOR TREATING CANCER", filed on November 17, 2017 at the China Patent Office, and the entire contents of which are incorporated herein by reference.

### FIELD

The present disclosure relates to a target for treating cancer. Specifically, the present disclosure relates to the use of the extracellular loop 1 (ECL1) of TM4SF1 as a target for treating cancer. More specifically, the present disclosure relates to an antibody capable of specifically binding to the extracellular loop 1 (ECL1) of TM4SF1, and the extracellular loop 1 (ECL1) peptide of TM4SF1.

### BACKGROUND

Angiogenesis involves the formation of new blood vessels from an existing network of blood vessels through proliferation, migration, and morphogenesis of vascular endothelial cells (EC). Convincing evidences show that the establishment of vascular network through angiogenesis is essential for the development of normal tissues and organs in the embryonic stage and the tissue proliferation process under pathological conditions. The vascular network provides a pathway for transport of oxygen and nutrient and removal of catabolites, and represents the rate-limiting step in most growth processes that occur in multicellular organisms. Therefore, it has been generally believed that vascular compartments are necessary not only for the development and differentiation of organs during embryogenesis, but also for the wounds healing and regeneration functions in an adult.

Angiogenesis is also implicated in the pathogenesis of a variety of diseases, including but not limited to: cancer, obesity, proliferative retinopathy, age-related macular degeneration, tumors, rosacea, atherosclerosis, rheumatoid arthritis (RA), cellular immunity and psoriasis. Angiogenesis is a cascade process consisting of degradation of the extracellular matrix at local sites after protease release, proliferation and migration of vascular endothelial cells. Since the significant physiological and pathological importance of angiogenesis, much work has been devoted to elucidating factors that can regulate this process.

TM4SF1 (Transmembrane-4L Six Family member 1), identified as an antigen of tumor cell in 1986, is a member of the L6 protein superfamily, a four-span transmembrane protein with two extracellular loops (Hellstrom et al Cancer Res. 46: 3917-3923, 1986). It was found that TM4SF1 is abundantly expressed on many cancer cells. Studies have also shown that TM4SF1 is only expressed in small amounts on vascular endothelial cells in normal tissues (DeNardo et al. Int J Rad Appl Instrum B.18: 621-631, 1991; Wright et al. Protein Sci. 9: 1594-1600, 2000; Richman et al. Cancer Res. 5916s-5920s, 1995; O'Donnell et al. Prostate. 37: 91-97, 1998), but highly expressed in vascular endothelial cells in tumor tissues (Shih et al. Cancer Res. 69: 3272-3277, 2009; Zukauskas et al. Angiogenesis. 14 : 345-354, 2011), retinal neovascular endothelial cells (English et al. J Biomed Inform. 42: 287-295, 2009), and vascular endothelial cells induced by VEGF-A (Shih et al. Cancer Res. 69: 3272-3277, 2009; Zukauskas et al. Angiogenesis. 14: 345-354, 2011).

Although the results of studies have shown that TM4SF1 can be used as a target of neovascularization to inhibit pathological angiogenesis and thus be used to treat pathological angiogenesis-related disorders, such as cancer, it is not known whether TM4SF1 can mediate tumor growth and metastasis through signal transduction. In particular, little is known about how TM4SF1 affects tumor cells at the molecular level. Therefore, it is necessary to explore treatment methods that target TM4SF1 on tumor cell surfaces and directly affect tumor cells, for example, to study new anti-TM4SF1 antibodies against tumor.

### SUMMARY

The inventors unexpectedly found in research that the extracellular loop 1 (ECL1) of TM4SF1 has antibody binding potential and may become a new therapeutic target. In particular, the inventors found that an antibody capable of specifically binding to the extracellular loop 1 (ECL1) of TM4SF1 can be a potential anticancer drug.

Based on the above findings, the present disclosure provides the use of an antibody capable of specifically binding to the extracellular loop 1 (ECL1) of TM4SF1 in the manufacture of a medicament for treating cancer.

In some embodiments, the antibody of the present disclosure is a chimeric antibody, a humanized antibody or a human antibody.

In some embodiments, the antibody of the present disclosure is a monoclonal antibody.

In some embodiments, the antibody of the present disclosure is a bispecific antibody.

In some embodiments, the antibody of the present disclosure is in the form of antibody-drug conjugate.

In some embodiments, the antibody is capable of specifically binding to an extracellular loop 1 (ECL1) peptide of TM4SF1, wherein the sequence of the ECL1 peptide is a consecutive fragment of SEQ ID NO: 2, and the fragment starts at position 27, 28, 29, 30, 31, 32 or 33 of SEQ ID NO: 2 and ends at position 42, 43, 44, 45, 46, 47 or 48 of SEQ ID NO: 2.

In some embodiments, the antibody of the present disclosure specifically binds the extracellular loop 1 (ECL1) peptide of TM4SF1 has a sequence set forth in SEQ ID NO: 4.

In some embodiments, the antibody of the present disclosure comprises a heavy chain and a light chain, wherein
(i) the heavy chain comprises three CDRs with respective sequences set forth in SEQ ID NO: 7, SEQ ID NO: 8 and SEQ ID NO: 9; and
(ii) the light chain comprises three CDRs with respective sequences set forth in SEQ ID NO: 10, SEQ ID NO: 11 and SEQ ID NO: 12.

In some embodiments, the cancer treated by the method of the present disclosure is selected from ovarian cancer, lung cancer, gastric cancer, breast cancer, liver cancer, pancreatic cancer, skin cancer, malignant melanoma, head and neck cancer, sarcoma, bile duct cancer, bladder cancer, kidney cancer, colon cancer, small intestine cancer, testicular cancer, placental chorionic cancer, cervical cancer, testicular cancer, uterine cancer, prostate cancer, multiple myeloma, malignant lymphoma, and metastases thereof.

In some embodiments, the cells of the cancer treated by the methods of the present disclosure express TM4SF1.

In another aspect, the present disclosure provides a method of treating cancer, comprising administering to a subject in need thereof a therapeutically effective amount of an antibody capable of specifically binding to the extracellular loop 1 (ECL1) of TM4SF1.

In yet another aspect, the present disclosure provides the use of the extracellular loop 1 (ECL1) peptide of TM4SF1 in the manufacture of a medicament for treating cancer. One available solution is to use the extracellular loop 1 (ECL1) peptide of TM4SF1 or a TM4SF1 recombinant protein containing the region to immunize animals to produce specific antibodies for the treatment of tumors and other diseases. In some embodiments, the extracellular loop 1 peptide of TM4SF1 or the TM4SF1 recombinant protein containing the region can be used for the screening of anti-TM4SF1 antibodies, for example, for the screening of hybridoma monoclonal antibodies and the screening of phage display antibody libraries. The extracellular loop 1 peptide of TM4SF1 or the TM4SF1 recombinant protein containing the region can also be used to determine the affinity of monoclonal antibodies, screen anti-TM4SF1 antibodies with neutralizing ability, and perform various cell tests and animal tests to screen, identify and validate antibodies or other molecular forms of drugs.

In some embodiments, the sequence of the extracellular loop 1 (ECL1) peptide of TM4SF1 of the present disclosure is a consecutive fragment of SEQ ID NO: 2, and the fragment starts at position 27, 28, 29, 30, 31, 32 or 33 of SEQ ID NO: 2 and ends at position 42, 43, 44, 45, 46, 47 or 48 of SEQ ID NO: 2.

In some embodiments, the extracellular loop 1 (ECL1) peptide of TM4SF1 of the present disclosure has a sequence set forth in SEQ ID NO: 4.

In yet another aspect, the present disclosure provides an isolated extracellular loop 1 (ECL1) peptide of TM4SF1 having a sequence which is a consecutive fragment of SEQ ID NO: 2, and the fragment starts at position 27, 28, 29, 30, 31, 32 or 33 of SEQ ID NO: 2 and ends at position 42, 43, 44, 45, 46, 47 or 48 of SEQ ID NO: 2.

In some embodiments, the peptide of the present disclosure has a tumor suppressing activity.

In some embodiments, the peptide of the present disclosure has a sequence set forth in SEQ ID NO: 4.

In yet another aspect, the present disclosure provides antibodies capable of specifically binding to the extracellular loop 1 (ECL1) of TM4SF1.

In some embodiments, the antibody of the present disclosure is a chimeric antibody, a humanized antibody, or a human antibody.

In some embodiments, the antibody of the present disclosure is a monoclonal antibody.

In some embodiments, the antibody of the present disclosure is a bispecific antibody.

In yet other embodiments, the antibodies of the present disclosure have an activity of inhibiting tumor, preferably suppressive activity on tumor metastasis, such as liver cancer, prostate cancer, lung cancer, colon cancer or metastases thereof.

In yet other embodiments, the antibodies of the present disclosure are used to inhibit tumors, preferably to inhibit tumor metastasis, such as liver cancer, prostate cancer, lung cancer, colon cancer or metastases thereof.

In some embodiments, the antibody of the present disclosure specifically binds to the extracellular loop 1 (ECL1) peptide of TM4SF1 of which the sequence is a consecutive fragment of SEQ ID NO: 2, and the fragment starts at position 27, 28, 29, 30, 31, 32 or 33 of SEQ ID NO: 2 and ends at position 42, 43, 44, 45, 46, 47 or 48 of SEQ ID NO: 2.

In some embodiments, the antibody capable of specifically binding to the extracellular loop 1 (ECL1) peptide of TM4SF1 has a sequence set forth in SEQ ID NO: 4.

In some embodiments, the antibody of the present disclosure comprises a heavy chain and a light chain, wherein
(i) the heavy chain comprises three CDRs with respective sequences set forth in SEQ ID NO: 7, SEQ ID NO: 8 and SEQ ID NO: 9; and
(ii) the light chain comprises three CDRs with respective sequences set forth in SEQ ID NO: 10, SEQ ID NO: 11 and SEQ ID NO: 12.

In yet another aspect, the present disclosure provides an antibody-drug conjugate comprising the antibody of the present disclosure and a therapeutic agent, wherein preferably the therapeutic agent is selected from the group consisting of a cytotoxic drug, an immune enhancer, and a radioisotope, more preferably the therapeutic agent is selected from the group consisting of aplysiatoxin and derivatives thereof, and most preferably, the therapeutic agent is selected from MMAE and MMAF.

In yet another aspect, the present disclosure provides a pharmaceutical composition comprising the ECL1 peptide of TM4SF1 of the present disclosure, the antibody of the present disclosure and/or the conjugate of the present disclosure, and a pharmaceutically acceptable carrier.

In another aspect, the present disclosure provides a conjugate comprising the antibody or functional fragment thereof of the present disclosure conjugated to one or more therapeutic agents, wherein preferably the therapeutic agent is cytotoxic drugs (e.g. antimetabolites, antitumor antibiotics, alkaloids), immune enhancers or radioisotopes; more preferably the therapeutic agent is selected from the group consisting of maytansinoids (e.g. Ansamitocin or Mertansine)), aplysiatoxin and derivatives thereof; most preferably the therapeutic agent is selected from the group consisting of MMAE (Monomethyl auristatin E) and MMAF (Monomethyl auristatin F). In other embodiments, the therapeutic agent may also be selected from the therapeutic agents listed in Table 1 below.

**Table 1 Therapeutic agents may be used in the conjugates of the present disclosure:**

| Abbreviation | Full Name | Type / Mechanism |
|---|---|---|
| MMAE | Monomethyl auristatin E | Microtubule monomer protein polymerization inhibitor |
| MMAE derivatives | | |
| MMAF | Monomethyl auristatin F | Microtubule monomer protein polymerization inhibitor |
| MMAF derivatives | | |
| DM1 | Mertansine derivative M4 | Microtubule depolymerization |
| DM4 | Mertansine derivative M4 | Microtubule depolymerization |
| Duocarmycine | Duocarmycine | DNA binding agent |
| Calicheamicin | Calicheamicin | DNA minor groove binding agent |
| PBDA | pyrrolobenzodiazepines | DNA binding agent |
| Doxorubicin | Doxorubicin | Topoisomerase inhibitor |
| Vinca Alkaloids | Vinca Alkaloids | |
| Metrotrexate | Metrotrexate | |
| Vinblastine | Vinblastine | Microtubule depolymerization |
| Daunorubincin | Daunorubincin | |

In some embodiments, the therapeutic agent is coupled to the antibody or functional fragment thereof via a linker. The linker used in the present disclosure may be linked to the antibody by any means known in the art, preferably via a thiol and/or amino group. In a particularly embodiment, the antibody of the present disclosure is linked to the linker via a thiol group. The linker used in the present disclosure may be a cleavable linker (that is, a linker that can be cleaved in *in vivo* environment) or a non-cleavable linker. In some embodiments, the linker of the present disclosure is selected from cleavable linkers, preferably from a peptide, hydrazone, and disulfide linker, such as maleimidocaproyl-valine-citrulline-p-aminobenzyloxycarbonyl (hereinafter abbreviated as mc-vc-pAB or vc). In other embodiments, the linker of the present disclosure is selected from non-cleavable linkers, such as maleimidocaproyl (hereinafter abbreviated as mc). In other embodiments, the linker may also be selected from those listed in Table 2 below.

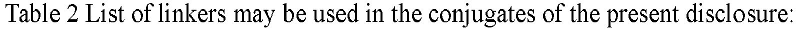

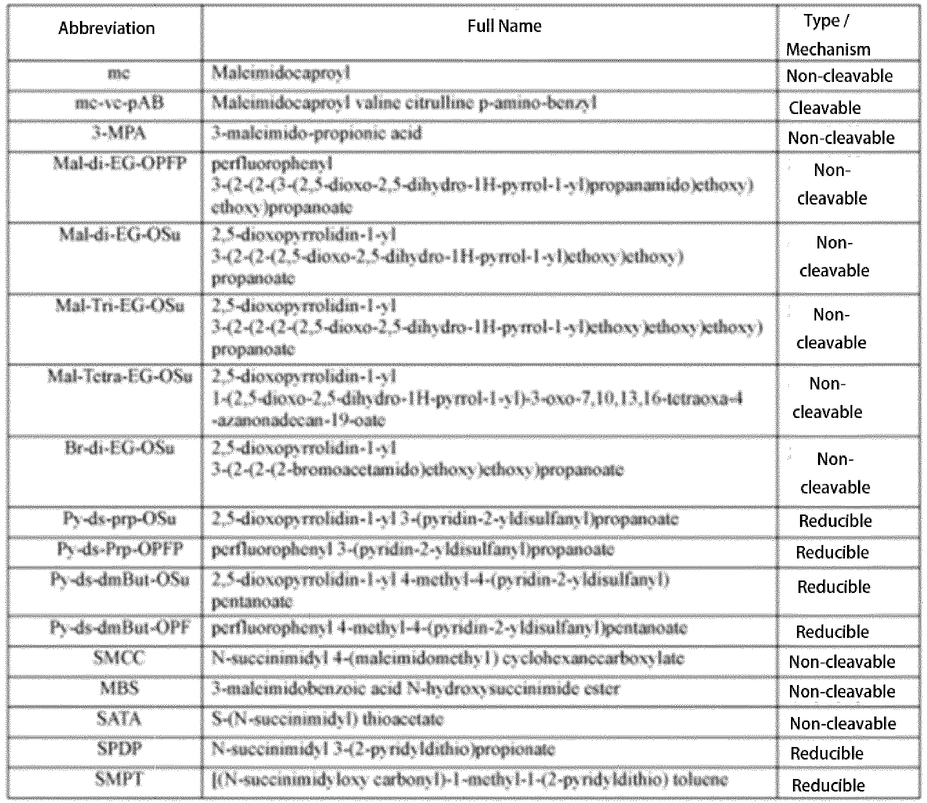

In another aspect, the present disclosure provides a conjugate having the general formula Ab-(L-U)ₙ, where Ab represents an antibody or a functional fragment thereof according to the present disclosure, and L represents a linker (e.g. mc-vc-pAB or mc), and U represents a therapeutic agent (preferably, the therapeutic agent is selected from the group consisting of cytotoxic drugs, immune enhancers, and radioisotopes, and more preferably, the therapeutic agent is selected from maytansinoids, aplysiatoxin and derivatives, and most preferably, the therapeutic agent is selected from MMAE and MMAF), and n is an integer from 1 to 8 (e.g., 1, 2, 3, 4, 5, 6, 7, or 8). The linker used in the present disclosure may be a cleavable linker (that is, a linker that can be cleaved in *in vivo* environment) or a non-cleavable linker.

### BRIEF DESCRIPTION OF THE DRAWINGS

Figure 1 shows the affinity between the monoclonal antibody FC17-7 of the present disclosure and TM4SF1.
Figure 2 shows that monoclonal antibody FC17-7 blocks the interaction between TM4SF1 and DDR1 in co-immunoprecipitation experiment.
Figure 3 is a Western Blotting image showing that P-STAT3 is down-regulated when the cells are treated with the monoclonal antibody FC17-7.
Figure 4 shows that the monoclonal antibody attenuates the formation of tumor cell spheres after the cells are treated with the monoclonal antibody FC17-7.
Figures 5 to 8 show that the monoclonal antibody significantly inhibits brain metastasis and bone metastasis of breast cancer and prolongs the survival time.
Figure 9 is a diagram showing that the small extracellular loop 1 peptide of TM4SF1 disrupts the interaction between TM4SF1 and DDR1.
Figure 10 shows that the small extracellular loop 1 peptide of TM4SF1 inhibits downstream signal transduction.
Figures 11 to 12 show that the small extracellular loop 1 peptide of TM4SF1 significantly inhibits metastasis of breast cancer.
Figure 13 shows that the small extracellular loop 1 peptide of TM4SF1 prolongs the survival mice.

### DETAILED DESCRIPTION

### Definitions

Unless defined otherwise, all technical and scientific terms used herein have the same meaning as understood by those skilled in the art. Regarding the definitions and terms in the art, professionals may refer to Current Protocols in Molecular Biology (Ausubel). Abbreviations for amino acid residues are standard 3-letter and/or 1-letter codes used in the art to refer to one of the 20 commonly used L-amino acids. In particular, the meaning of the terms used herein can also be found in Chinese patent application NO. 201110131029.X.

Although the numerical ranges and parameter approximations shown in the broad scope of the present disclosure, the numerical values shown in the specific examples are described as accurately as possible. However, any numerical value inherently must contain a certain amount of error, which is caused by the standard deviation present in their respective measurements. In addition, all ranges disclosed herein are understood to cover any and all subranges contained therein. For example, a range of "1 to 10" should be considered including any and all subranges between a minimum of 1 and a maximum of 10 (inclusive); that is, all subranges beginning with a minimum of 1 or greater, such as 1 to 6.1, and subranges ending with a maximum of 10 or less, such as 5.5 to 10. In addition, any reference referred to as "incorporated herein" is to be understood as being incorporated in its entirety.

It should also be noted that, as used in this specification, the singular form includes the plural form of the object to which it refers, unless it is clearly and explicitly limited to one object. The term "or" is used interchangeably with the term "and/or" unless the context clearly indicates otherwise.

The term "soluble" protein as used herein refers to a protein that is soluble in aqueous solution at biologically relevant temperatures, pH levels and osmotic pressures. In certain embodiments, the fusion protein of the present disclosure is a soluble protein. "Soluble fusion protein" as used herein means that the fusion protein does not include transmembrane domain and intracellular domain.

As used herein, the term "isolated" refers to the substances and/or entities that (1) are separated from at least some of the components with which they were originally generated (in the natural environment and/or in the experimental setting) and/or (2) are produced, prepared and/or manufactured manually. Isolated substances and/or entities can be separated from at least about 10%, about 20%, about 30%, about 40%, about 50%, about 60%, about 70%, about 80%, about 90%, about 95%, about 98%, about 99%, substantially 100%, or 100% of the other components with which are was originally associated. In certain embodiments, the fusion protein of the present disclosure is an isolated fusion protein.

The terms "portion" and "fragment" are used interchangeably to refer to a part of a peptide, nucleic acid, or other molecular construct.

The term "subject" as used herein refers to mammals, such as humans, and other animals, such as domestic animals (such as dogs, cats, etc.), farm animals (such as cattle, sheep, pigs, horses, etc.) or experimental animals (such as monkeys, rats, mice, rabbits, guinea pigs, etc.).

The terms "identity", "percent identity" and "homology" as used herein refer to sequence identity between two amino acid sequences or between nucleic acid sequences. Percent identity can be determined by aligning two sequences. Percent identity refers to the number of same residues (i.e., amino acids or nucleotides) at the common position shared by the sequences being compared. Sequence alignments and comparisons can be performed using standard algorithms in the art (e.g. Smith and Waterman, 1981, Adv. Appl. Math. 2: 482; Needleman and Wunsch, 1970, J. Mol. Biol. 48: 443; Pearson and Lipman, 1988, Proc. Natl .Acad.Sci., USA, 85: 2444) or computerized versions of these algorithms (Wisconsin Genetics Software Package Release 7.0, Genetics Computer Group, 575 Science Drive, Madison, WI), which are publicly available as BLAST and FASTA. In addition, ENTREZ available from the National Institutes of Health (Bethesda MD) can be used for sequence comparisons. When using BLAST and gap BLAST programs, the default parameters of each program (e.g., BLASTN, available on the Internet site of the National Center for Biotechnology Information) can be used. In one embodiment, a GCG with a gap weight of 1 can be used to determine the percent identity of two sequences such that each amino acid gap is weighted as if there is a single amino acid mismatch between the two sequences. Alternatively, the ALIGN program (version 2.0) can be used, which is a part of the GCG (Accelrys, San Diego, CA) sequence alignment software package.

In some embodiments, the methods of the present disclosure can be used alone. Alternatively, the methods can be used in combination with other conventional anti-cancer therapies for the treatment or prevention of proliferative diseases (such as tumors). For example, these methods can be used to prevent cancer, prevent cancer recurrence and postoperative metastasis, and may be used as an adjuvant way to other cancer treatments. The present disclosure demonstrates that the effectiveness of conventional cancer treatments (e.g., chemotherapy, radiotherapy, phototherapy, immunotherapy, and surgery) can be enhanced by using target peptide therapeutics.

As used herein, the terms "pharmaceutical composition," "combined drug," and "drug combination" are used interchangeably and mean a combination of at least one drug and optionally a pharmaceutically acceptable carrier or excipient used to achieve a specific purpose. In certain embodiments, the pharmaceutical composition includes combinations that are separated in time and/or space as long as they can work together to achieve the purpose of the present disclosure. For example, the ingredients contained in the pharmaceutical composition (such as an antibody, a nucleic acid molecule, a combination of nucleic acid molecules, and/or a conjugate according to the present disclosure) may be administered to a subject as a whole or separately. When the ingredients contained in the pharmaceutical composition are separately administered to a subject, the ingredients may be administered to the subject simultaneously or sequentially. Preferably, the pharmaceutically acceptable carrier is water, a buffered aqueous solution, an isotonic saline solution such as PBS (phosphate buffered saline), glucose, mannitol, dextrose, lactose, starch, magnesium stearate, cellulose, magnesium carbonate, 0.3% glycerol, hyaluronic acid, ethanol or polyalkylene glycols such as polypropylene glycol, triglycerides and the like. The type of the pharmaceutically acceptable carrier used depends in particular on whether the composition according to the present disclosure is formulated for oral, nasal, intradermal, subcutaneous, intramuscular or intravenous administration. The composition according to the present disclosure may contain wetting agents, emulsifiers or buffer substances as additives.

In some embodiments, cancers that can be treated with the antibodies disclosed herein include but not limited to, for example primary mesenchymal tumors (sarcomas), fibrosarcomas, myxosarcomatous, liposarcomas, chondrosarcoma, osteosarcoma, angiosarcoma, endotheliosarcoma, lymphangiosarcoma, synoviosarcoma, mesothelioma, Ewing's tumor, granulocytic leukemia, monocytic leukemia, malignant leukemia, lymphatic leukemia, plasmacytoma, leiomyosarcoma, and rhabdomyosarcoma, primary epithelial cancer (carcinoma), squamous cell or epidermal carcinoma, basal cell carcinoma, sweat gland carcinoma, sebaceous gland carcinoma, adenocarcinoma, papillary carcinoma, papillary adenocarcinoma, cystadenocarcinoma, medullary carcinoma, undifferentiated carcinoma (simple carcinoma), bronchogenic carcinoma, bronchopulmonary carcinoma, melanoma, renal cell carcinoma, hepatocellular carcinoma, bile duct carcinoma, transitional cell carcinoma, squamous cell carcinoma, chorionic carcinoma, seminoma, embryonic carcinoma, malignant teratoma, teratoma, leukemia, acute lymphocytic leukemia and acute myeloid leukemia (myeloid, promyelocytic, myeloid monocyte; monocytic and erythrocyte leukemia), chronic leukemia, chronic myeloid (granulocytic) leukemia, chronic lymphocytic leukemia, polycythemia vera, lymphoma, Hodgkin's disease, non-Hodgkin's disease, multiple myeloma, primary macroglobulinemia, and heavy chain disease. In some preferred embodiments, the cancer is selected from ovarian cancer, lung cancer, gastric cancer, breast cancer, liver cancer, pancreatic cancer, skin cancer, malignant melanoma, head and neck cancer, sarcoma, bile duct cancer, bladder cancer, kidney cancer, colon cancer, small intestine cancer, testicular cancer, placental chorionic cancer, cervical cancer, testicular cancer, uterine cancer, prostate cancer, ovarian cancer, and metastases thereof.

The pharmaceutical composition, vaccine or pharmaceutical formulation according to the present disclosure can be administered by any suitable route, for example, orally, nasally, intradermally, subcutaneously, intramuscularly or intravenously.

The term "therapeutic agent" as used herein means any substance or entity having a therapeutic effect (e.g., treating, preventing, alleviating or inhibiting any disease and/or disorder), including but not limited to: chemotherapeutic agents, radiotherapeutics, immunotherapeutics, thermally therapeutic agents, and the like.

As used herein, "CDR region" or "CDR" refers to the hypervariable regions of the heavy and light chains of an immunoglobulin, as defined by Kabat et al. (Kabat et al.,Sequences of proteins of immunological interest,5th Ed.,U.S.Department of Health and Human Services,NIH,1991, and later). There are three heavy chain CDRs and three light chain CDRs. As used herein, the term CDR or CDRs is used to indicate one or several or even all of these regions, which contain most of the amino acid residues responsible for the binding through the affinity between an antibody and an antigen or epitope thereof.

For the purposes of the present disclosure, the "identity", "consistency" or "similarity" between two nucleic acid or amino acid sequences refers to the percentage of identical nucleotides or identical amino acid residues between two sequences to be compared obtained after optimal alignment. The percentage is purely statistical and the differences between the two sequences are randomly distributed over their full length. Sequence comparisons between two nucleic acid or amino acid sequences are usually performed by comparing these sequences after they have been optimally matched, and the comparison can be performed on a segment or on a "comparison window". In addition to manual implementation, the optimal alignment for comparing sequences can also be performed by Smith and Waterman (1981)[Ad.App.Math.2:482] local homology algorithm, by Neddleman and Wunsch (1970)[J.MoI.Biol.48:443] local homology algorithm, by Pearson and Lipman (1988)[Proc.Natl.Acad.Sci.USA 85:2444) similarity search method, and by computer software using these algorithms (GAP, BESTFIT, FASTA and TFASTA in the Wisconsin Genetics Software Package, Genetics Computer Group, 575 Science Dr., Madison, WI, or by the BLAST N or BLAST P comparison software).

As used herein, a "therapeutically effective amount" or "effective amount" refers to a dose sufficient to exert its benefit to the subject being administered. The actual amount administered, as well as the rate and time course of administration, will depend on the condition of the person being treated and the severity. The prescription of treatment (e.g., dose determination, etc.) is ultimately the responsibility and decision of the GP and other doctors, usually considering the disease to be treated, the individual patient's condition, the delivery site, the method of administration, and other factors known to the doctor.

The term "subject" as used herein refers to mammals, such as humans, but can also be other animals, such as wild animals (such as herons, storks, cranes, etc.), farm animals (such as ducks, geese, etc.) or experimental animals (such as orangutan, monkey, rat, mouse, rabbit, guinea pig, groundhog, ground squirrel, etc.).

The term "antibody" refers to a whole antibody and any antigen-binding fragment ("antigen-binding portion") or single chain thereof. A "full-length antibody" refers to a protein comprising at least two heavy (H) chains and two light (L) chains interconnected by disulfide bonds. Each heavy chain contains a heavy chain variable region (abbreviated as VH) and a heavy chain constant region. The heavy chain constant region contains three domains, CH1, CH2, and CH3. Each light chain contains a light chain variable region (abbreviated as VL) and a light chain constant region. The light chain constant region contains a domain, CL. The VH and VL regions can be further subdivided into multiple regions with high variability, called complementarity determining regions (CDRs), interspersed with more conservative regions called frame regions (FRs). Each VH and VL is composed of three CDRs and four FRs, which are arranged from the amino terminal to the carboxy terminal in the following order: FR1, CDR1, FR2, CDR2, FR3, CDR3, FR4. These variable regions of the heavy and light chains contain binding domains that interact with the antigen. The constant region of an antibody can mediate binding of immunoglobulin to the host tissues or factors, including various cells of the immune system (such as effector cells) and the first component of the classical complement system (Clq). Chimeric or humanized antibodies are also encompassed by the antibodies according to the present disclosure.

The term "humanized antibody" refers to an antibody that contains a CDR region derived from a non-human antibody and the rest of the antibody molecule is derived from one (or several) human antibodies. Moreover, in order to retain binding affinity, some residues of the backbone (called FR) segment can be modified (Jones et al., Nature, 321: 522-525, 1986; Verhoeyen et al., Science, 239: 1534-1536, 1988; Riechmann et al., Nature, 332: 323-327, 1988). Humanized antibodies or fragments thereof according to the present disclosure can be prepared by techniques known to those skilled in the art (e.g., described in the document Singer et al., J. Immun. 150: 2844-2857, 1992; Mountain et al., Biotechnol. Genet. Eng. Rev., 10: 1-142, 1992; or Bebbington et al., Bio/Technology, 10: 169-175, 1992).

The term "chimeric antibody" refers to an antibody in which the variable region sequence is from one species and the constant region sequence is from another species, for example, an antibody in which the variable region sequence is from a mouse antibody and the constant region sequence is from a human antibody. A chimeric antibody or a fragment thereof according to the present disclosure can be prepared by using genetic recombination technology. For example, the chimeric antibody can be produced by cloning a recombinant DNA comprising a promoter and a sequence encoding a variable region of a non-human, especially a murine monoclonal antibody according to the present disclosure, and a sequence encoding a constant region of a human antibody. The chimeric antibody of the present disclosure encoded by such a recombinant gene will be, for example, a mouse-human chimera whose specificity is determined by the variable region derived from mouse DNA, and the isotype is determined by the constant region derived from human DNA. For methods for preparing a chimeric antibody, for example, reference can be made to the document Verhoeyn et al. (BioEssays, 8:74, 1988).

The term "monoclonal antibody" refers to a preparation of antibody molecules consisting of single molecules. Monoclonal antibody compositions display a single binding specificity and affinity for a particular epitope.

The term "derivative" refers to a (chemical) modification of an amino acid/amino acid chain at the N-terminus, C-terminus, backbone, peptide bond, and/or side chain residue. The term is not intended to refer to any additions, substitutions or deletions of amino acids in the amino acid chain. (Chemical) derivatives from such L-amino acids or L-enantiomeric amino acids generally include any natural or non-naturally occurring derivative of these amino acids, including, but not limited to: the amino acids as defined above, which comprise post-translational modifications or synthetic modifications, including acetylation (at the N-terminus of the (poly)peptide sequence, at a lysine residue, etc.), deacetylation, alkylation such as methylation, ethylation, etc. (preferably at a lysine or arginine residue in the (poly) peptide sequence), dealkylation such as demethylation, deethylation, etc., amidation (preferably at the C-terminus of the (poly)peptide sequence), formylation, γ-carboxylation, glutamylation, glycosylation (preferably at a asparagine, lysine, hydroxylysine, serine or threonine residues in the (poly) peptide sequence, etc.), increase of heme or heme moiety, hydroxylation, iodination, isoprenoid or increase of isoprenoid moiety such as farnesyl or geranylgeraniol, etc.), lipoylation (linking via lipoic acid functional groups) such as isoprenylation, GPI anchor formation, including myristoylation, farnesylation, geranylgernaylation, etc., oxidation, phosphorylation (e.g., to a serine, tyrosine, threonine or histidine moiety in the (poly)peptide sequence), sulfuric acid (e.g., sulfation of tyrosine), selenoylation, sulfation, and the like.

### Administration

The fusion protein in the present disclosure may be administered alone, but preferably administered as a pharmaceutical composition, which usually includes a suitable pharmaceutical excipient, diluent, or carrier selected according to the planned manner of administration. The fusion proteins can be administered to patients in need of treatment by any suitable means. The accurate dose will depend on a number of factors, including the precise nature of the fusion protein.

Some suitable administration manners include, but not limited to, oral, rectal, nasal, topical (including buccal and sublingual), subcutaneous, vaginal or parenteral (including subcutaneous, intramuscular, intravenous, intradermal, intrathecal, and epidural) administration.

For intravenous injections and injections at the focus, the active ingredient will be in the form of a parenterally acceptable aqueous solution, which contains no heat source and has a suitable pH, isotonicity and stability.

Those skilled in the art can use appropriate solvents or formulations to formulate the fusion protein, for example: isotonic excipients such as sodium chloride injection, Ringer's injection, and lactated Ringer's injection. As required, preservatives, stabilizers, buffers, antioxidants and/or other additives can be added. Pharmaceutical compositions for oral administration may be in the form of tablets, capsules, powders, or oral solutions. A tablet may include a solid carrier such as gelatin or an adjuvant. Liquid pharmaceutical compositions typically include a liquid carrier such as water, petroleum, animal or vegetable oils, mineral oils or synthetic oils. A physiological saline solution, glucose or other sugar solutions or glycols such as ethylene glycol, propylene glycol or polyethylene glycol may also be included.

Examples of the techniques and protocols mentioned above, as well as other techniques and protocols used in accordance with the present disclosure, can be found in Remington's Pharmaceutical Sciences, 16th edition, Oslo, A. (ed), 1980.

### Examples

### Example 1. Preparation of anti-TM4SF1 monoclonal antibody

In order to prepare a monoclonal antibody with high affinity to TM4SF1, a peptide having a sequence of FPNGETKYASENHLSC was designed and synthesized according to the amino acid sequence of extracellular loop 1 of TM4SF1. BALB/c mice (Shanghai SLAC Experimental Animal Center) were immunized with the TM4SF1 peptide, and the spleen lymphocytes of the immunized mice were collected and fused with SP2/0 myeloma cells (purchased from ATCC) to prepare hybridomas cells. The fused cells were cultured in a 96-well cell culture plate for cloning. The TM4SF1 peptide-coated ELISA plate was used to detect anti-TM4SF1 positive clones by ELISA to obtain several anti-TM4SF1 hybridoma cell lines. The hybridoma cells of positive clone were expanded and subcloned. As a result, several anti-TM4SF1 hybridoma cell lines were obtained. The antibodies showed high affinity for TM4SF1 in ELISA and flow cytometry assay. Finally, several monoclonal antibodies such as clone FC17-7 were identified to have high affinity to the target. The FC17-7 hybridoma cell line has been deposited in CHINA CENTER FOR TYPE CULTURE COLLECTION (CCTCC), material name: anti-TM4SF1 antibody (FC17-7), accession number CCTCC NO: C2017110, deposit date: July 13, 2017. In order to obtain the sequence of the FC17-7 monoclonal antibody, total RNA was extracted from the FC17-7 monoclonal antibody hybridoma cell line and subjected to reverse transcription to synthesize cDNA. The heavy chain and light chain variable region DNA sequences of the anti-TM4SF1 antibody were amplified by PCR to determine the amino acid sequences of the variable regions of the heavy chain and light chain, and the sequence of the six CDR regions of the heavy chain and light chain of the antibody were identify by sequence analysis (amino acid sequences are SEQ ID No: 7, SEQ ID No: 8 and SEQ ID No: 9; SEQ ID No: 10, SEQ ID No: 11 and SEQ ID No: 12).

### Example 2. Binding assay between the monoclonal antibody and TM4SF1

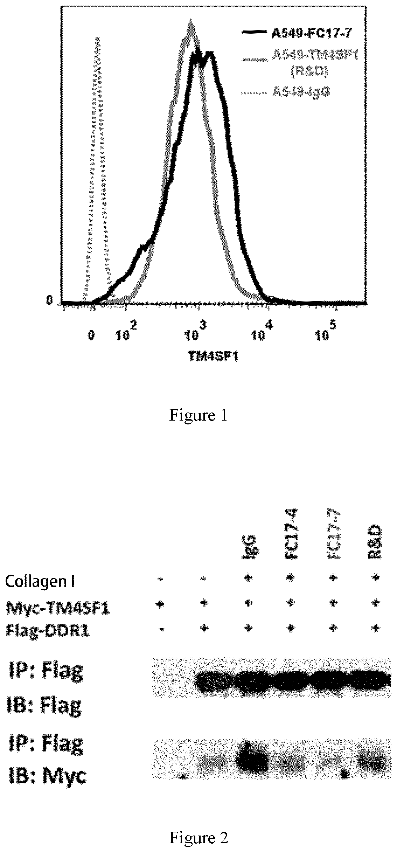

A549 lung cancer cell line with high expression of TM4SF1 was cultured in DMEM-HG medium. After the cell confluency reached 90% or more, the medium was discarded, and the cells were washed once with PBS, digested with 0.25% trypsin at 37 °C in an incubator for 5 minutes, and then the digestion was terminated by the same volume of culture medium. After centrifugation at 1,000 rpm for 5 min, the supernatant was removed, and the cells were resuspended in PBS containing 10% FBS, and 1% sodium azide. The cells were counted and divided into different tubes, resulting in 1×10⁶ cells per tube. After centrifugation at 1,000 rpm for 5 minutes, the supernatant was removed, and 100 µl of PBS containing 3% BSA and the monoclonal antibody (primary antibody) prepared in Example 1 at desired concentrations were added to each sample tube, incubating for 30 minutes. Washing: each sample was resuspended in PBS, and centrifuged at 1,000 rpm for 5min. This step was repeated three times in total. 100 µl of PBS containing 3% BSA was added to each sample, and then APC-conjugated anti-mouse secondary antibody at a dilution of 1:500 was added, incubating for 30 min. Each sample was resuspended in PBS containing 3% BSA and 1% sodium azide, centrifuged at 1,000 rpm for 5 min. This step was repeated three times. Finally, the sample cells were resuspended in 200 µl PBS, and detected by flow cytometry. The results showed that the FC17-7 antibody can specifically bind to TM4SF1. Live cell FACS experiments demonstrated that the FC17-7 monoclonal antibody can efficiently bind to TM4SF1 on the surface of cell membranes with high affinity (Figure 1).

### Example 3. Preparation of TM4SF1 peptide

In order to suppress TM4SF1 signaling pathway, a soluble peptide having the sequence of FPNGETKYASENHLSRC was designed based on the sequence of extracellular loop 1 of TM4SF1. The peptide contains 17 amino acids and was synthesized by Shanghai Gill Biochemical Company. The synthesized peptide passed the quality control and was used for cell and animal experiments.

### Example 4. Monoclonal antibody blocks the interaction of TM4SF1 and DDR1

To show that the monoclonal antibody can block the interaction of TM4SF1 and DDR1, 293FT cells were used to co-overexpress TM4SF1 and DDR1. 293FT cells were cultured in DMEM-HG medium and digested with trypsin. The cells were counted and seeded at a density of 1 × 10⁶ cells per dish in 6 cm (diameter) culture dish. The next day, 700 µl of serum-free DMEM-HG medium was respectively added to two centrifuge tubes. 10 µl of P-Pei transfection reagent was added to the first centrifuge tube. 1 µg PQCXIP-hTM4SF1 plasmid and 1 µg PQCXIN-hDDR1 plasmid were added to the second centrifuge tube and mixed well. The resultant from the second centrifuge tube was added to the first centrifuge tube and incubated for 20 min to obtain the transfection mixture. The culture medium of the 293FT cells seeded the day before was removed, and the transfection mixture above was added to the culture dish and incubated for 6 hours. Fresh medium was added to continue the culture. 48 hours after the transfection, the medium was removed, 2 ml of serum-free DMEM-HG medium containing a certain concentration of FC17-7 antibody obtained in Example 1 or IgG (as a negative control) was added. After incubating the culture dish at 37 °C for 5 minutes, Collagen I was added to the culture dish to a final concentration of 10 µg/ml. The cells were cultured for 6 hours and then collected. The cells were lysed with IP-RIPA buffer in a refrigerator at 4 °C for 1 h, then centrifuged at 12,000 rpm for 30 min. The supernatant was sampled to determine the protein concentration, and the protein concentration of each sample was adjusted to be the same. Anti-flag M2 affinity gel was added to each sample. After 2 hours incubation at 4 °C, the supernatant was discarded by centrifugation at 3,000 rpm for 2 minutes, and the pellet was washed with IP-RIPA. This step was repeated three times. Elution buffer containing 3 µl Flag peptide (5 µg /ml) was added to the Anti-flag M2 affinity gel and incubated on ice for 1 h. After the elution, the mixture was centrifuged at 3,000 rpm for 2 min to collect the supernatant, and the protein expression was detected by Western Blotting. The results demonstrated that TM4SF1 interacts with DDR1 at their extracellular regions, co-immunoprecipitation experiments showed that FC17-7 antibody not only binds extracellular loop of TM4SF1 but also blocks the interaction between TM4SF1 and DDR1 (Figure 2).

### Example 5. Monoclonal antibody blocks TM4SF1 associated non-classical DDR1 signaling pathway

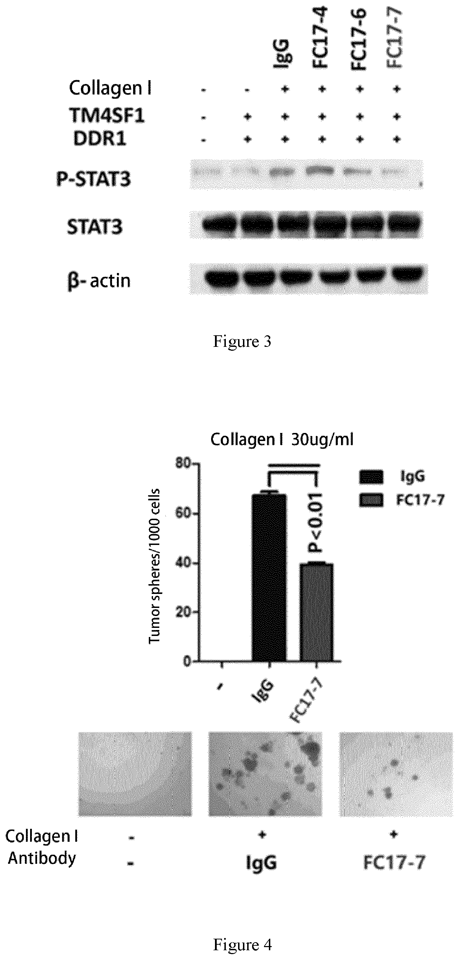

In order to show that the signal pathway of TM4SF1 can be blocked by the monoclonal antibody, TM4SF1 and DDR1 were overexpressed in cells. The transfection method has been described in Example 4. 48 hours after transfection, the medium was removed, 2 ml of serum-free DMEM-HG medium containing a certain concentration of FC17-7 antibody obtained in Example 1 or IgG (as a negative control) was added. After incubating the culture dish at 37 °C for 5 minutes, Collagen I was added to the culture dish to a final concentration of 10 µg/ml and cultured for 12 hours. The cells were collected and lysed with IP-RIPA buffer in a refrigerator at 4 °C for 1 h. The lysate was sonicated twice (15s 30% power), then centrifuged at 12,000 rpm for 30 min. The supernatant was sampled to determine the protein concentration, and the protein concentration of each sample was adjusted to be the same. The sample was subjected to SDS-PAGE electrophoresis, and the proteins were transferred to a membrane by conventional way in Western Blotting, and then stained with the antibody. Experiment results show that when TM4SF1 activates the non-classical DDR1 signal pathway, the downstream p-STAT3 level will increase, which will affect downstream functions. Western Blotting experiments of this example demonstrated that when FC17-7 antibody was used to treat the cells, the downstream signal transduction of non-classical DDR1 signaling pathway associated with TM4SF1 was inhibited and p-STAT3 level was down-regulated (Figure 3).

### Example 6: Monoclonal antibody inhibits the formation of tumor cell spheres

MDAMB231-BoM-1833-TGL cells were digested into single cell suspension, and seeded into a low attachment 24-well plate at 1,000 cells per well in a MEBM medium containing B27 (1:50), 5mg/ml insulin, 0.5mg/ml hydrocortisone, 20ng/ml bFGF, 20ng/ml EGF, 4mg/ml heparin, 30µg/ml Collagen I and 10µg/ml FC17-7 antibody of Example 1 or IgG (as a negative control). After the cells were cultured for 7 days, the number of cell spheres formed from the tumor cells was counted under a microscope. The experimental results show that TM4SF1 interacts with DDR1 and activates downstream signaling pathways, so that enhances the expressions of the downstream Sox2, Oct4, Nanog and other stem cell transcription factors, thereby promoting the proliferation and invasion of tumor cells. The sphere formation assay usually reflects the proliferation and invasion ability of a tumor. When the cells were treated with FC17-7 antibody, the formation of tumor cell spheres was significantly reduced, indicating that the monoclonal antibody can inhibit the proliferation of tumor cells (Figure 4).

### Example 7. Monoclonal antibody or peptide inhibits tumor cell metastasis in mice in vivo experiments

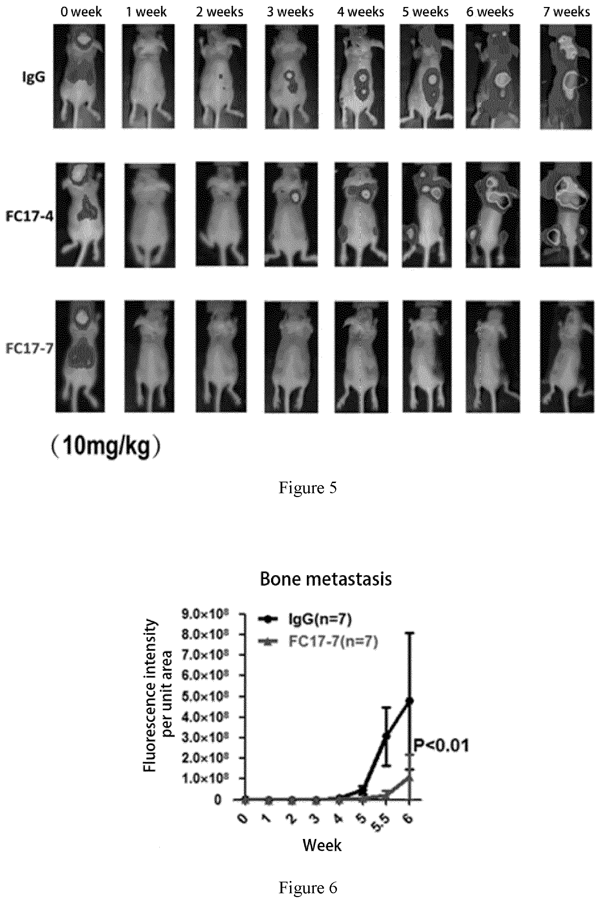

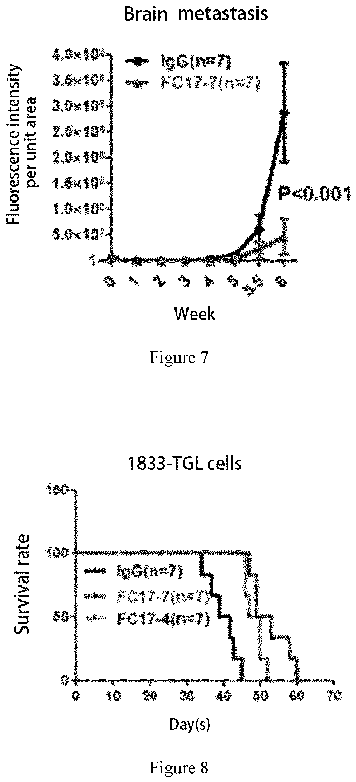

The day before the injection of tumor cells into mice was recorded as Day -1 of the experiment. On Day -1, the mice were fasted for 4 hours, injected with the desired concentration of FC17-7 antibody obtained in Example 1 or IgG (as a negative control), and given food after 2 hours. After the first injection, the mice were injected with antibodies every three days. On Day 0 of the experiment, MDAMB231-BoM-1833-TGL cells were blocked with antibodies according to the method of Example 4. After 6 hours, the cells were digested. The medium was removed, and the cells were washed with PBS once and resuspended in PBS to a concentration of 1 × 10⁶ cells/ml. Then, mice were injected with 1 × 10⁵ tumor cells /100 µl by ventricular injection to establish a mouse metastasis model. Every other week, the mice were subjected to in vivo imaging to record the fluorescence signal data. By establishing a breast cancer metastasis model, periodically administration of the monoclonal antibody FC17-7 prepared in Example 1 was performed in mice. It was found that FC17-7 antibody can significantly inhibit the metastasis of breast cancer to various organs, including brain and bone (Figure 5 to 7); meanwhile, the monoclonal antibody prolongs the survival time of mice (Figure 8).

### Example 8. Effect of extracellular loop 1 peptide of TM4SF1 on tumor metastasis - blocking the interaction of TM4SF1 and DDR1

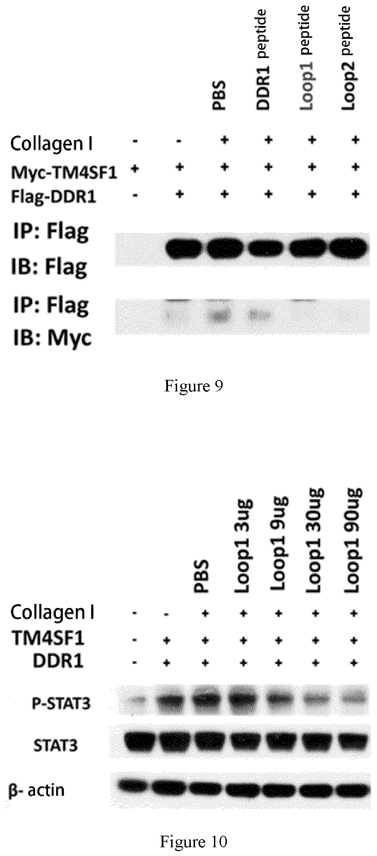

First, 293FT cells were used to overexpress the extracellular loop 1 peptide of TM4SF1, extracellular loop 2 peptide of TM4SF1, or DDR1 extracellular peptide, respectively. The cells were lysed with IP-RIPA buffer in a refrigerator at 4 °C for 1 h, and then centrifuged at 12,000 rpm for 30 min. The supernatant was sampled to determine the protein concentration, and the protein concentration of each sample was adjusted to be the same. Anti-flag M2 affinity gel beads were added to each sample. After 2 hours incubation at 4 °C, the supernatant was discarded by centrifugation at 3,000 rpm for 2 minutes, and the pellet was washed with IP-RIPA. This step was repeated three times. Elution buffer containing 3 µl Flag peptide (5 µg /ml) was added to the Anti-flag M2 affinity gel and incubated on ice for 1 h. After the elution, the mixture was centrifuged at 3,000 rpm for 2 min to collect the supernatant. Then, 293FT cells were used to co-overexpress TM4SF1 and DDR1. The transfection method has been described in Example 4. 48 hours after transfection, the medium was removed, and 2 ml of serum-free DMEM-HG medium containing the extracellular loop 1 peptide of TM4SF1, extracellular loop 2 peptide of TM4SF1, or DDR1 extracellular peptide prepared in this example or IgG (as a negative control) was added. After incubating the culture dish at 37 °C for 5 minutes, Collagen I was added to the culture dish to a final concentration of 10 µg/ml. The cells were cultured for 6 hours and then collected for co-immunoprecipitation. For the co-immunoprecipitation experiment and the subsequent Western Blotting experiment, refer to the methods in Example 4. The experimental results show that by treating cells with extracellular loop 1 peptide of TM4SF1, extracellular loop 2 peptide of TM4SF1 or DDR1 extracellular peptide, it was found that the extracellular loop 1 peptide of TM4SF1 can significantly disrupt the interaction between TM4SF1 and DDR1 (Figure 9).

### Example 9. Effect of the extracellular loop 1 peptide of TM4SF1 on tumor metastasis - inhibiting TM4SF1 associated non-classical DDR1 signaling pathway

In order to show that the TM4SF1 signaling pathway can be blocked by the extracellular loop 1 peptide of TM4SF1, TM4SF1 and DDR1 were overexpressed in cells. The transfection method has been described in Example 4. 48 hours after transfection, the medium was removed, 2 ml of serum-free DMEM-HG medium containing a certain concentration of the extracellular loop 1 peptide of TM4SF1 prepared in Example 3 or PBS (as a negative control) was added. After incubating the culture dish at 37 °C for 5 minutes, Collagen I was added to the culture dish to a final concentration of 10 µg/ml. The cells were cultured for 12 hours and then collected. The cells were lysed with IP-RIPA buffer in a refrigerator at 4 °C for 1 h. The lysate was sonicated twice (15s 30% power), and then centrifuged at 12,000 rpm for 30 min. The supernatant was sampled to determine the protein concentration, and the protein concentration of each sample was adjusted to be the same. The sample was subjected to SDS-PAGE electrophoresis, and the proteins were transferred to a membrane by a conventional way used in Western Blotting, and then stained with antibody. The experimental results show that after treating the cells with different concentrations of the extracellular loop 1 peptide of TM4SF1, at a concentration of 9 µg/ml, the extracellular loop 1 peptide of TM4SF1 began to inhibit P-STAT3 which is a downstream of TM4SF1 associated non-classical DDR1 signaling pathway in a dose-dependent manner (Figure 10).

### Example 10. Effect of the extracellular loop 1 peptide of TM4SF1 on tumor metastasis - inhibiting metastasis of breast cancer

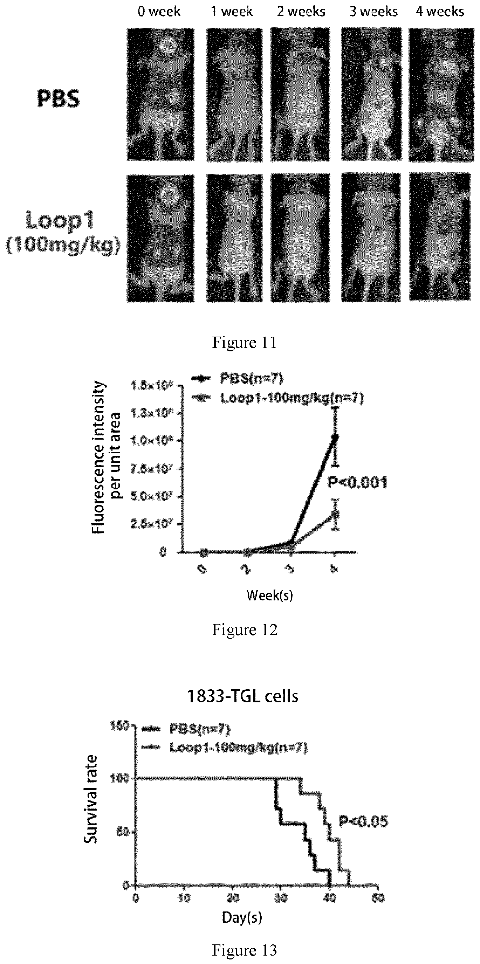

The day before the injection of tumor cells into mice was recorded as Day -1 of the experiment. On Day -1, the mice were fasted for 4 hours, and injected with the desired concentration of the extracellular loop 1 peptide of TM4SF1 prepared in Example 3 or IgG (as negative control), and given food after 2 hours. After the first injection of the peptide, the mice were injected with the peptide every other day. On Day 0 of the experiment, MDAMB231-BoM-1833-TGL cells were blocked with the peptide according to the method of Example 9. After 6 hours, the cells were digested. The medium was removed, and the cells were washed with PBS once, and resuspended in PBS to a concentration of 1 × 10⁶ cells/ml. For the method of establishing a breast cancer metastasis mouse model, refer to the method in Example 7. The experimental results show that by establishing a breast cancer metastasis model, and periodically treating the mice with the extracellular loop 1 peptide of TM4SF1, it was found that the peptide can significantly inhibit the metastasis of breast cancer (Figures 11 and 12), and can prolong the survival time of the mice (Figure 13).

The present disclosure has been exemplified by various specific embodiments. However, those of ordinary skill in the art can understand that the present disclosure is not limited to the specific embodiments. Those of ordinary skill in the art can make various changes or modifications within the scope of the present disclosure, and various technical features mentioned in various places in this specification can be combined with each other without departing from the spirit and scope of the present disclosure. Such modifications and variations are all within the scope of the present disclosure.

## Claims

1. Use of an antibody capable of specifically binding to extracellular loop 1 (ECL1) of TM4SF1 for the manufacture of a medicament for treating cancer.

2. The use according to claim 1, wherein the antibody is a chimeric antibody, a humanized antibody or a human antibody.

3. The use according to claim 1, wherein the antibody is a monoclonal antibody.

4. The use according to claim 1, wherein the antibody is a bispecific antibody.

5. The use according to claim 1, wherein the antibody is in the form of antibody-drug conjugate.

6. The use according to claim 1, wherein the antibody is capable of specifically binding to an extracellular loop 1 (ECL1) peptide of TM4SF1, and wherein the sequence of the ECL1 peptide is a consecutive fragment of SEQ ID NO: 2, and the fragment starts at position 27, 28, 29, 30, 31, 32 or 33 of SEQ ID NO: 2 and ends at position 42, 43, 44, 45, 46, 47 or 48 of SEQ ID NO: 2.

7. The use according to claim 1, wherein the antibody is capable of specifically binding to the extracellular loop 1 (ECL1) peptide of TM4SF1 having the sequence set forth in SEQ ID NO: 4.

8. The use according to claim 1, wherein the antibody comprises a heavy chain and a light chain, and wherein
(i) the heavy chain comprises three CDRs with respective sequences set forth in SEQ ID NO: 7, SEQ ID NO: 8 and SEQ ID NO: 9; and
(ii) the light chain comprises three CDRs with respective sequences set forth in SEQ ID NO: 10, SEQ ID NO: 11 and SEQ ID NO: 12.

9. The use according to claim 1, wherein the cancer is selected from the group consisting of ovarian cancer, lung cancer, gastric cancer, breast cancer, liver cancer, pancreatic cancer, skin cancer, malignant melanoma, head and neck cancer, sarcoma, bile duct cancer, bladder cancer, kidney cancer, colon cancer, small intestine cancer, testicular cancer, placental chorionic cancer, cervical cancer, testicular cancer, uterine cancer, prostate cancer, multiple myeloma, malignant lymphoma, and metastases thereof.

10. The use according to claim 1, wherein cells of the cancer express TM4SF1.

11. Use of the extracellular loop 1 (ECL1) peptide of TM4SF1 for the manufacture of a medicament for treating cancer.

12. The use according to claim 11, wherein the sequence of the ECL1 peptide is a consecutive fragment of SEQ ID NO: 2, and the fragment starts at position 27, 28, 29, 30, 31, 32 or 33 of SEQ ID NO: 2 and ends at position 42, 43, 44, 45, 46, 47 or 48 of SEQ ID NO: 2.

13. The use according to claim 11, wherein the ECL1 peptide has a sequence set forth in SEQ ID NO: 4.

14. The use according to claim 11, wherein the cancer is selected from the group consisting of ovarian cancer, lung cancer, gastric cancer, breast cancer, liver cancer, pancreatic cancer, skin cancer, malignant melanoma, head and neck cancer, sarcoma, bile duct cancer, bladder cancer, kidney cancer, colon cancer, small intestine cancer, testicular cancer, placental chorionic cancer, cervical cancer, testicular cancer, uterine cancer, prostate cancer, multiple myeloma, malignant lymphoma, and metastases thereof.

15. The use according to claim 11, wherein cells of the cancer express TM4SF1.

16. An isolated extracellular loop 1 (ECL1) peptide of TM4SF1, wherein the sequence of the peptide is a consecutive fragment of SEQ ID NO: 2, which starts at position 27, 28, 29, 30, 31, 32 or 33 of SEQ ID NO: 2 and ends at position 42, 43, 44, 45, 46, 47 or 48 of SEQ ID NO: 2.

17. The peptide according to claim 16, wherein the peptide has an activity of inhibiting tumor.

18. The peptide according to claim 16, which has a sequence set forth in SEQ ID NO: 4.

19. An antibody capable of specifically binding to extracellular loop 1 (ECL1) of TM4SF1.

20. The antibody according to claim 19, which is a chimeric antibody, a humanized antibody or a human antibody.

21. The antibody according to claim 19, which is a monoclonal antibody.

22. The antibody according to claim 19, which is a bispecific antibody.

23. The antibody of claim 19, which is capable of specifically binding to the extracellular loop 1 (ECL1) peptide of TM4SF1, wherein the sequence of the ECL1 peptide is a consecutive fragment of SEQ ID NO: 2, which starts at position 27, 28, 29, 30, 31, 32 or 33 of SEQ ID NO: 2 and ends at position 42, 43, 44, 45, 46, 47 or 48 of SEQ ID NO: 2.

24. The antibody of claim 19, which is capable of specifically binding to the extracellular loop 1 (ECL1) peptide of TM4SF1 having a sequence set forth in SEQ ID NO: 4.

25. The antibody according to claim 19, which comprises a heavy chain and a light chain, wherein
(i) the heavy chain comprises three CDRs with respective sequences set forth in SEQ ID NO: 7, SEQ ID NO: 8 and SEQ ID NO: 9; and
(ii) the light chain comprises three CDRs with respective sequences set forth in SEQ ID NO: 10, SEQ ID NO: 11 and SEQ ID NO: 12.

26. The antibody according to claim 19, which is secreted by hybridoma cells deposited at the China Center for Type Culture Collection with an accession number CCTCC NO: C2017110 on July 13, 2017.

27. An antibody-drug conjugate comprising the antibody according to any one of claims 19-26 and a therapeutic agent,
preferably, the therapeutic agent is selected from the group consisting of a cytotoxic drug, an immune enhancer and a radioisotope,
more preferably, the therapeutic agent is selected from the group consisting of aplysiatoxin and derivative thereof, and
most preferably, the therapeutic agent is selected from the group consisting of MMAE and MMAF.

28. A pharmaceutical composition comprising the peptide according to any one of claims 16 to 18, the antibody according to any one of claims 19 to 26 and/or the conjugate according to claim 27, together with a pharmaceutically acceptable carrier.

29. A method for screening a cancer drug, comprising
(i) contacting a test substance with the extracellular loop 1 (ECL1) of TM4SF1; and
(ii) determining whether the test substance specifically binds to the extracellular loop 1 (ECL1) of TM4SF1;
wherein the specific binding of the test substance to the extracellular loop 1 (ECL1) of TM4SF1 indicates that the test substance can be used as a cancer drug.
